# EUROPÄISCHE PATENTSCHRIFT

(11) **EP 2 366 338 B1**
(45) Veröffentlichungstag und Bekanntmachung des Hinweises auf die Patenterteilung: **25.10.2017**
(21) Anmeldenummer: 11001967.6
(22) Anmeldetag: 10.03.2011
(51) Int. Cl.: A61B 17/06, A61B 17/04, A61B 17/062

(54) **Laparoskopischer Nadelhalter**
Laparoscopic needle holder
Support d'aiguille laparoscopique

(30) Priorität: 18.03.2010 DE 102010011926
(43) Veröffentlichungstag der Anmeldung: 21.09.2011
(73) Patentinhaber: OLYMPUS WINTER & IBE GMBH, 22045 Hamburg (DE)
(72) Erfinder: Hirschfeld, Simon, 22303 Hamburg (DE); Bauer, Markus, 20539 Hamburg (DE)
(74) Vertreter: Hausfeld, Norbert

(56) Entgegenhaltungen:
- EP-A1- 0 507 622
- EP-A2- 2 044 892
- WO-A2-2008/113076
- DE-A1-102006 034 590

## Beschreibung

Die Erfindung betrifft einen laparoskopischen Nadelhalter der im Oberbegriff des Anspruchs 1 genannten Art, ein Verfahren zu dessen Herstellung nach Anspruch 5 und einen gebogenen distalen Schaftabschnitt des Nadelhalters nach Anspruch 7.

Laparoskopische Nadelhalter sind in der heute typischen Standardbauweise in US 5,413,583 A sowie US 5,951,587 A dargestellt. Sie weisen am distalen Ende eines langgestreckten dünnen Schaftrohres ein Zangenmaul auf, mit dem chirurgische Nadeln gehalten und zum Zwecke des Nähens bewegt werden können. Der Handgriff ist bei den bekannten Instrumenten typischerweise als Inline-Griff ausgebildet, also mit einer Erstreckung im Wesentlichen in Richtung des Schaftrohres. Diese Griffform ist sowohl zum Ausüben der erforderlichen hohen Haltekräfte als auch für die typische Nähbewegung gut geeignet. Mit dem langgestreckten Schaft eignen sich gattungsgemäße Instrumente zum Einführen durch einen laparoskopischen Port in den Bauchraum, um dort unter endoskopischer Beobachtung nähen zu können.

In jüngster Zeit setzt sich eine laparoskopische Technik durch, wie sie in der DE 202009007592 U1 dargestellt ist. Dabei wird mit zwei Instrumenten durch einen Port gearbeitet. Aus den in dieser Schrift erläuterten Gründen ist es dabei vorteilhaft, wenn die Schäfte der Instrumente in ihrem distalen Abschnitt gebogen ausgeführt sind, da dann die Instrumente Spitze an Spitze arbeiten, dennoch aber im Bereich des gemeinsamen Ports parallel aneinander liegen können.

WO 2008/113076 A2 beschreibt einen laparoskopischen Nadelhalter mit einem distalen gebogenen Schaftabschnitt, der drehbar an einem proximalen Schaftabschnitt befestigt ist.

EP 2 044 892 A2 beschreibt einen medizinischen Wundhefter an einer flexiblen Welle, wobei die Welle lösbar an einen proximalen Handgriff befestigbar ist.

Aus EP 0 507 622 A1 ist eine laparoskopische Zange mit einem distal an einem Schaft angeordneten Maulteil bekannt, das mit einer in dem Schaft verschiebbar angeordneten Betätigungsstange bewegungsgekoppelt ist. An ihrem proximalen Ende weist die Betätigungsstage ein Mitnehmerelement auf, das zur Zug-/Druckbelastung mit einem beweglichen Griffelement eines Handgriffes verbunden ist.

Die Aufgabe der vorliegenden Erfindung besteht darin, einen laparoskopischen Nadelhalter auf kostengünstige Weise für die Verwendung in einem gemeinsam genutzten Port auszubilden.

Diese Aufgabe wird mit den Merkmalen der Ansprüche 1, 5 und 7 gelöst.

Erfindungsgemäß ist der Nadelhalter mit einem Schaft ausgebildet, der einen gebogenen distalen Schaftabschnitt und einen geraden proximalen Schaftabschnitt aufweist, also für die aus der letztgenannten Schrift bekannten Zwecke hervorragend geeignet ist. Die beiden Schaftteile sind lösbar aneinander befestigt und können daher getrennt angefertigt werden. Das vereinfacht und verbilligt die Herstellung außerordentlich, da der gerade proximale Schaftabschnitt, zusammen mit dem Handgriff, von einem gattungsgemäßen Nadelhalter übernommen werden kann. Lediglich der gebogene distale Schaftabschnitt muss gesondert angefertigt werden, wobei sich die Probleme aufgrund der geringen Länge dieses Schaftabschnittes verringern.

Die beiden Schaftabschnitte sind erfindungsgemäß über eine Kupplung verbunden, die zur Aufnahme eines Zangenmaules ausgebildet ist. Daher kann alternativ am distalen Ende des geraden proximalen Schaftabschnittes unmittelbar ein Zangenmaul angeordnet sein. Die Einheit aus geradem proximalem Schaftabschnitt und Handgriff kann also sowohl zusammen mit einem Zangenmaul als gerader Nadelhalter nach dem Stand der Technik verwendet werden, als auch mit angekuppeltem gebogenem distalem Schaftabschnitt als Nadelhalter zur Verwendung mit anderen Instrumenten in einem gemeinsamen Port.

Das Zangenmaul kann am distalen Ende des distalen Schaftabschnittes auf unterschiedliche Weise befestigt sein, beispielsweise zu Reinigungszwecken abnehmbar oder auch drehbar. Vorteilhaft kann das Zangenmaul jedoch am distalen Abschnitt dauerhaft befestigt, z.B. verschweißt, wodurch die Konstruktion und die Herstellung erheblich vereinfacht werden.

Am proximalen Ende der Stange ist in an sich bekannter Weise ein verdickter Mitnehmer, z.B. in der üblichen in Form einer Kugel, vorgesehen, der zur Kupplung mit den bewegten Teilen des Handgriffes dient. Dieser Mitnehmer kann auf unterschiedliche Weise, z.B. mittels Verschweißung, am Ende der Stange befestigt sein, ist dabei aber vorteilhaft in individuell eingestelltem Abstand befestigt, womit Längenunterschiede der Stange aufgefangen werden können, die sonst die exakte Anpassung der Bewegungsbereiche des Zangenmaules und des Handgriffes stören würden.

Vorteilhaft ist hierbei der Mitnehmer verschraubt und verschweißt. Dies ermöglicht zunächst eine Längenverstellung mit der Verschraubung, um anschließend mittels Verschweißung die Verschraubung festsetzen zu können.

Erfindungsgemäß ist zudem ein Verfahren zur Herstellung des erfindungsgemäßen Nadelhalters, wonach zunächst Zangenmaul und Stange verbunden werden. Sodann wird die Stange in den Schaft eingeführt und das Zangenmaul am distalen Ende des distalen Schaftteiles befestigt. Dieses ist dabei noch gerade und wird erst anschließend, zusammen mit der innenliegenden Stange, gebogen. Danach wird der distale Schaftabschnitt am proximalen Schaftabschnitt befestigt. Diese Schrittabfolge hat sich besonders bewährt, da sie bei niedrigen Kosten eine hohe Fertigungsqualität sichert.

Vorteilhaft wird der Mitnehmer, der üblicherweise als Kugel ausgebildet ist, im gewünschten Abstand an der Stange durch Verschrauben eingestellt und durch anschließendes Schweißen befestigt. Auch diese Schrittfolge hat sich als besonders vorteilhaft und kostengünstig herausgestellt.

Erfindungsgemäß ist zudem eine den gebogenen distalen Schaftabschnitt eines laparoskopischen Nadelhalters enthaltende Baugruppe.

In der Zeichnung ist die Erfindung beispielsweise und schematisch dargestellt. Es zeigen:
- Fig. 1: eine Seitenansicht eines erfindungsgemäßen Nadelhalters,
- Fig. 2: eine Ansicht entsprechend Fig. 1 nur der den distalen Schaftabschnitt des Nadelhalters der Fig. 1 enthaltenden Baugruppe und
- Fig. 3: eine Seitenansicht einer anstelle der Konstruktion der Fig. 2 verwendbaren Baugruppe.

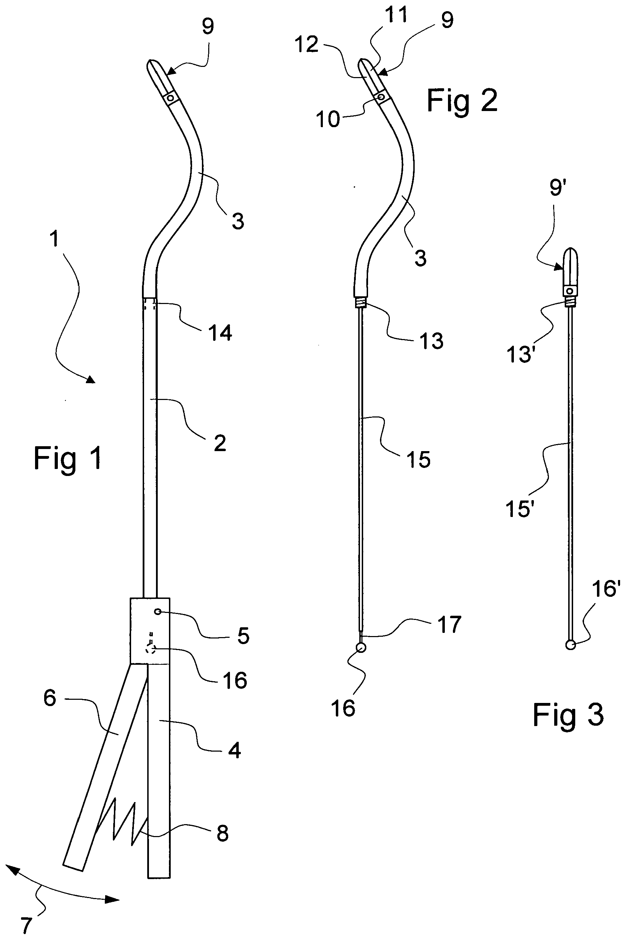

Fig. 1 zeigt einen kompletten erfindungsgemäßen Nadelhalter 1 in Seitenansicht. Der Nadelhalter 1 weist einen als Rohr ausgebildeten Schaft auf, mit einem geraden proximalen Schaftabschnitt 2 und einem gebogenen distalen Schaftabschnitt 3.

Am proximalen Ende des proximalen Schaftabschnittes 2 ist ein Handgriff 4 befestigt, der als feststehendes Griffstück ausgebildet ist und an dem um eine Achse 5 im Sinne des Pfeils 7 schwenkbar ein bewegliches Griffstück 6 gelagert ist, das gegenüber dem feststehenden Handgriff 4 mit einer Feder 8 abgestützt ist. Bei dieser Konstruktion liegt die typische Form eines Inline-Griffes vor, der für Nadelhalter besonders geeignet ist. Es kann jedoch auch eine andere Griffart verwendet werden, wie beispielsweise ein Pistolen-Handgriff.

Der distale Schaftabschnitt 3 des Schaftes 2, 3 ist in Fig. 2 gesondert dargestellt. Man erkennt am distalen Ende des distalen Schaftabschnittes 3 ein Zangenmaul 9, das mit zwei um eine Achse 10 schwenkbar gelagerten Maulteilen 11, 12 ausgebildet ist.

Am proximalen Ende des distalen Schaftabschnittes 3 ist ein Verbindungselement in Form eines Gewindestückes 13 mit Außengewinde angeordnet, das zum Einschrauben in ein Innengewinde 14 am distalen Ende des proximalen Schaftabschnittes 2 dient.

Eine Stange 15 ist am Zangenmaul 9 zu dessen Betätigung befestigt, durchläuft den rohrförmigen distalen Schaftabschnitt 3 und überragt diesen in distaler Richtung, wie dies in Fig. 2 dargestellt ist. Die Stange 15 ist an ihrem proximalen Ende mit einem Mitnehmer in Form einer Kugel 16 versehen, die mit einem Gewindestück 17 in das distale Ende der Stange 15 einschraubbar ist.

Die in Fig. 2 dargestellte Baugruppe lässt sich, wie Fig. 1 zeigt, an das distale Ende des proximalen Schaftabschnittes 2 des Schaftes ansetzen und durch Einschrauben des Gewindestückes 13 in das Innengewinde 14 fest verkuppeln. Dabei wird das proximal überragende Ende der Stange 15 mit der Kugel 16 in proximaler Richtung durch den proximalen Schaftabschnitt 2 des Schaftes geschoben, bis die Kugel 16 in der in Fig. 1 gestrichelt dargestellten Stellung steht, in der sie in nicht dargestellter Weise mit dem internen Betätigungsmechanismus des Handgriffes 4 gekuppelt ist, und zwar üblicherweise in der Art, dass beim Zusammendrücken der Stücke 4 und 6 des Handgriffes 4 die Stange 15 in proximaler Richtung gezogen wird, woraus sich eine Schließbewegung der Maulteile 11 und 12 ergibt.

In den Figuren 1 und 2 sind aus Gründen der übersichtlichen Darstellung das Zangenmaul 9 und der Handgriff 4 derart am Schaft 2, 3 angeordnet, dass sie in der Zeichnungsebene öffnen und schließen. Die entsprechenden Achsen 10 und 5 stehen dabei senkrecht zur Zeichnungsebene. Aus Gründen besserer Handhabung kann es vorteilhaft sein, das Zangenmaul 9 und/oder den Handgriff 4 unter anderen Winkeln am Schaft anzuordnen, z.B. gegenüber der dargestellten Stellung um 90° gedreht.

Ist die im Wesentlichen aus dem distalen Schaftabschnitt 3 und der Stange 15 bestehende, in Fig. 2 dargestellte, Baugruppe abgenommen, so kann an ihrer Stelle eine andere Baugruppe in Form eines in Fig. 3 dargestellten Zangenmaules 9' mit einer daran angeordneten Stange 15' mit Kugel 16' an dem proximalen Schaftabschnitt 2 des Nadelhalters 1 befestigt werden. Das Zangenmaul 9' entspricht dem Zangenmaul 9 der Konstruktion der Fig. 2. An ihm ist ein Gewindestück 13' befestigt, das dem Gewindestück 13 der Fig. 2 entspricht. Die Baugruppe der Fig. 3 lässt sich also anstelle der Baugruppe der Fig. 2 am Zangenhalter 1 anschrauben, wobei die Kugel 16' dann anstelle der Kugel 16 mit dem Handgriff 4, 6 gekuppelt ist. Damit lässt sich die in Fig. 1 dargestellte Zange, die mit ihrem gebogenen distalen Schaftabschnitt 3 für das Arbeiten mit mehreren Instrumenten in einem gemeinsamen laparoskopischen Port ausgebildet ist, auf eine konventionelle Nadelhalterkonstruktion umrüsten, bei der das Zangenmaul 9' unmittelbar am distalen Ende des geraden proximalen Schaftabschnittes 2 sitzt.

An der Stange 15' der Konstruktion der Fig. 3 ist die Kugel 16' direkt befestigt, z.B. verschweißt. In Serienherstellung können diese Bauteile alle mit konstanter Länge der Stange 15' und somit mit konstantem Abstand der Kugel 16' zum Zangenmaul 9' hergestellt werden. Diese Bauteile passen dann stets zu dem proximalen Schaftabschnitt 2 des Nadelhalters 1.

Bei der Konstruktion der Fig. 2 durchläuft die Stange 15 jedoch den gebogenen distalen Schaftabschnitt 3, bei dem erhebliche Fertigungstoleranzen auftreten können. Daher ist hier das Gewindestück 17 von Vorteil, mit dem der Abstand der Kugel 16 zum Gewindestück 13 auf das genau zum proximalen Schaftabschnitt 2 des Nadelhalters 1 passenden Maß eingestellt werden kann, um anschließend verschweißt zu werden.

Der Nadelhalter 1, entsprechend der Konfiguration der Fig. 1 und 2, wird wie folgt hergestellt:
Der gerade proximale Schaftabschnitt 2 des Schaftes wird zusammen mit dem Handgriff 4, 5, 6, 8 vorgefertigt. Vorzugsweise kann er aus einer Bauserie entnommen werden, die zusammen mit dem Einsatz der Fig. 3 als konventioneller gerader Nadelhalter gefertigt ist.

Der gebogene distale Schaftbereich, der in der Fig. 2 dargestellt ist, wird getrennt vorgefertigt. Das Zangenmaul 9 kann ebenfalls aus der Serie entnommen werden, aus der auch das Zangenmaul 9' der Fig. 3 stammt. Am Zangenmaul 9 wird zunächst die Stange 15 befestigt. Diese wird sodann durch ein zunächst noch gerades Rohrstück gesteckt, an dessen distalem Ende das Zangenmaul 9 z.B. durch Verschweißung befestigt wird. Sodann wird dieses Rohrstück, zusammen mit der darin liegenden Stange 15 zu dem gebogenen distalen Schaftabschnitt 3 gebogen. Am distalen Ende der Stange 15 befindet sich ein Gewindeloch, in das das Gewindestück 17 mit der Kugel 16 eingeschraubt wird, bis die Kugel 16 zu dem proximalen Ende des distalen Schaftabschnittes 3 den gewünschten Abstand hat. Dann wird das Gewindestück 17 verschweißt, um die Kugel 16 in ihrem Abstand dauerhaft festzulegen.

Anschließend wird die so gebildete, in Fig. 2 dargestellte Baugruppe im Nadelhalter 1 der Fig. 1 eingebaut, und zwar genauso, wie die Baugruppe der Fig. 3, durch Einschrauben am Innengewinde des proximalen Schaftabschnittes 2 und durch Kuppeln der Kugel 16 im Handgriff 4.

Anstelle der Befestigung der Schaftabschnitte 2 und 3 des Schaftes aneinander mit den Gewindeteilen 13 und 14 kann hier auch eine andere lösbare Kupplung vorgesehen sein, z.B. mittels Bajonett oder dergleichen. Dabei ist aber dafür Sorge zu tragen, dass die Winkelstellung des gebogenen distalen Schaftabschnittes 3 des Schaftes zum Handgriff 4 stets korrekt ausgerichtet ist.

Das Zangenmaul 9 ist bei der dargestellten Konstruktion der Fig. 1 und 2 mit dem distalen Schaftabschnitt 3 des Schaftes drehfest verbunden, z.B. verschweißt. Daraus ergibt sich eine sehr einfache und kostengünstige Konstruktion. Zur Vereinfachung des Nähvorganges wäre es jedoch durchaus wünschenswert, das Zangenmaul 9 gegenüber dem distalen Schaftabschnitt 3 verdrehen zu können. Dann wäre dort eine entsprechende Drehlagerung erforderlich und eine Drehübertragung vom Handgriff 4 bis zum Zangenmaul 9 könnte z.B. durch die drehsteife Stange 15 erfolgen. Am Handgriff 4 müsste dann ein Drehsteller vorgesehen sein, der an der Stange 15 entsprechend angreift.

## Patentansprüche

1. Laparoskopischer Nadelhalter (1) mit einem als Rohr ausgebildeten Schaft (2, 3), an dessen distalem Ende ein Zangenmaul (9) angeordnet ist, das von einer den Schaft (2, 3) durchlaufenden Stange (15) betätigbar ist, welche von einem am proximalen Ende des Schaftes (2, 3) angeordneten Handgriff (4) längsverschiebbar ist, wobei der Schaft (2, 3) einen geraden proximalen Schaftabschnitt (2) und einen gebogenen distalen Schaftabschnitt (3) aufweist, wobei die beiden Schaftabschnitte (2, 3) lösbar aneinander befestigt sind, und wobei die Schaftabschnitte (2, 3) über eine Kupplung (14) am distalen Ende des proximalen Schaftabschnittes (2) verbunden sind, **dadurch gekennzeichnet, dass** die Kupplung (14) zur Aufnahme eines dem Zangenmaul (9) entsprechenden Zangenmaules (9') ausgebildet ist.

2. Nadelhalter nach einem der vorhergehenden Ansprüche, **dadurch gekennzeichnet, dass** das Zangenmaul (9) am distalen Schaftabschnitt (3) nichtlösbar befestigt ist.

3. Nadelhalter nach einem der vorhergehenden Ansprüche, **dadurch gekennzeichnet, dass** die Stange (15) an ihrem proximalen Ende einen verdickten Mitnehmer (16) zur Kupplung mit dem Handgriff (4) aufweist, wobei der Mitnehmer (16) in individuell eingestelltem Abstand an der Stange (15) befestigbar ist.

4. Nadelhalter nach Anspruch 3, **dadurch gekennzeichnet, dass** der Mitnehmer (16) verschraubt und verschweißt ist.

5. Verfahren zur Herstellung eines Nadelhalters (1) nach einem der vorhergehenden Ansprüche, **dadurch gekennzeichnet, dass** eine vorgefertigte Montageeinheit aus Zangenmaul (9) und Stange (15) mit der Stange (15) in den distalen Schaftabschnitt (3) eingeführt wird, dass dann das Zangenmaul (9) am distalen Ende des distalen Schaftabschnittes (2) befestigt wird, dass dann der distale Schaftabschnitt (3) mit innenliegender Stange (15) gebogen wird, und dass schließlich der distale Schaftabschnitt (3) am proximalen Schaftabschnitt (2) mittels einer zur Aufnahme eines dem Zangenmaul (9) entsprechenden Zangenmaules (9') ausgebildeten Kupplung (14) befestigt wird.

6. Verfahren nach Anspruch 6, **dadurch gekennzeichnet, dass** der Mitnehmer (16) im gewünschten Abstand durch Verschrauben eingestellt und dann durch Schweißen befestigt wird.

7. Baugruppe eines laparoskopischen Nadelhalters (1), mit einem als Rohr ausgebildeten distalen Schaftabschnitt (3) an dessen distalem Ende ein Zangenmaul (9) angeordnet ist, das von einer den distalen Schaftabschnitt (3) durchlaufenden Stange (15) betätigbar ist, wobei der distale Schaftabschnitt (3) gebogen ausgebildet ist und an seinem proximalen Ende ein Verbindungselement (13) zur Verbindung mit dem distalen Ende eines proximalen Schaftabschnittes (2) des Nadelhalters (1) aufweist, **dadurch gekennzeichnet, dass** die Schaftabschnitte (2, 3) über eine Kupplung (14) am distalen Ende des proximalen Schaftabschnittes (2) verbunden sind, die zur Aufnahme eines dem Zangenmaul (9) entsprechenden Zangenmaules (9') ausgebildet ist, und wobei an dem das Verbindungselement (13) proximal überragenden Ende der Stange (15) ein verdickter Mitnehmer (16) angeordnet ist.

## Claims

1. Laparoscopic needle holder (1) with a stem (2, 3) that is designed in the form of a tube at the distal end of which a beak of forceps (9) is arranged that can be actuated by a rod (15) that extends through the stem (2, 3), whereby the rod can be shifted in longitudinal direction by a handle (4) that is arranged on the proximal end of the stem (2, 3), whereby the stem (2, 3) comprises a straight proximal stem section (2) and a curved distal stem section (3), whereby the two stem sections (2, 3) are fastened to each other in detachable manner, and whereby the stem sections (2, 3) are connected by means of a coupling (14) on the distal end of the proximal stem section (2), **characterised in that** the coupling (14) is designed to accommodate a beak of forceps (9') that matches the beak of forceps (9).

2. Needle holder according to any one of the preceding claims, **characterizsd in that** the beak of forceps (9) is fastened to the distal stem section (3) in non-detachable manner.

3. Needle holder according to any one of the preceding claims, **characterised in that** the rod (15) comprises, on its proximal end, a thickened driver (16) for coupling to the handle (4), whereby the driver (16) can be fastened to the rod (15) at an individually adjusted distance.

4. Needle holder according to claim 3, **characterised in that** the driver (16) is screw-retained and welded.

5. Method for the production of a needle holder (1) according to any one of the preceding claims, **characterised in that** a pre-manufactured assembly unit made of beak of forceps (9) and rod (15) is inserted, by means of the rod (15), into the distal stem section (3), **in that** the beak of forceps (9) is then fastened to the distal end of the distal stem section (2), **in that** the distal stem section (3) is then curved while the rod (15) is situated inside it, and **in that** lastly the distal stem section (3) is attached to the proximal stem section (2) by means of a coupling (14) that is designed to accommodate a beak of forceps (9') that matches the beak of forceps (9).

6. Method according to claim 6, **characterised in that** the driver (16) is adjusted to the desired distance by screw-retention and is then fastened by welding.

7. Assembly unit of a laparoscopic needle holder (1) with a stem section (3) that is designed in the form of a tube at the distal end of which a beak of forceps (9) is arranged that can be actuated by a rod (15) that extends through the distal stem section (3), whereby the distal stem section (3) is designed to be curved and comprises, on its proximal end, a connecting element (13) for connection to the distal end of a proximal stem section (2) of the needle holder (1), **characterised in that** the stem sections (2, 3) are connected by means of a coupling (14) on the distal end of the proximal stem section (2), whereby the coupling (14) is designed to accommodate a beak of forceps (9') that matches the beak of forceps (9), and whereby a thickened driver (16) is arranged on the end of the rod (15) that projects beyond the connecting element (13) in proximal direction.

## Revendications

1. Porte-aiguille laparoscopique (1) avec une gaine (2, 3) de conformation tubulaire à l'extrémité distale de laquelle est agencé un mors de pince (9) pouvant être actionné par une tige (15) traversant la gaine (2, 3), laquelle tige (15) peut être déplacée longitudinalement par une poignée (4) agencée à l'extrémité proximale de la gaine (2, 3), la gaine (2, 3) présentant une section de gaine proximale rectiligne (2) et une section de gaine distale incurvée (3), les deux sections de gaine (2, 3) étant fixées l'une à l'autre de façon amovible et les sections de gaine (2, 3) étant reliées par un accouplement (14) à l'extrémité distale de la section de gaine proximale (2), **caractérisé en ce que** l'accouplement (14) est conformé de façon à pouvoir recevoir un mors (9') correspondant au mors (9).

2. Porte-aiguille selon l'une des revendications précédentes, **caractérisé en ce que** le mors de pince (9) est fixé à la section de gaine distale (3) de façon non amovible.

3. Porte-aiguille selon l'une des revendications précédentes, **caractérisé en ce que** la tige (15) présente à son extrémité proximale un entraîneur (16) plus gros assurant l'accouplement à la poignée (4), l'entraîneur (16) pouvant être fixé à la tige (15) avec un écartement individuellement réglable.

4. Porte-aiguille selon la revendication 3, **caractérisé en ce que** l'entraîneur (16) est vissé et soudé.

5. Procédé de fabrication d'un porte-aiguille (1) selon l'une des revendications précédentes, **caractérisé en ce qu'**une unité de montage préfabriquée comportant un mors (9) et une tige (15) est introduite avec la tige (15) dans la section de gaine distale (3) et **en ce que** le mors (9) est alors fixé à l'extrémité distale de la section de gaine distale (2) et que la section de gaine distale (3) avec la tige (15) qui y est logée est incurvée et qu'enfin la section de gaine distale (3) est fixée à la section de gaine proximale (2) au moyen d'un accouplement (14) conformé de façon à pouvoir recevoir un mors de pince (9') correspondant au mors (9).

6. Procédé selon la revendication 6, **caractérisé en ce que** l'entraîneur (16) est réglé sur l'écartement voulu par vissage puis fixé par soudage.

7. Module de porte-aiguille laparoscopique (1), avec une section de gaine distale (3) de conformation tubulaire à l'extrémité distale de laquelle est agencé un mors de pince (9) pouvant être actionné par une tige (15) traversant la section de gaine distale (3), la section de gaine distale (3) étant de conformation incurvée et présentant à son extrémité proximale un élément d'assemblage (13) pour l'assemblage avec l'extrémité distale d'une section de gaine proximale (2) du porte-aiguille (1), **caractérisé en ce que** les sections de gaine (2, 3) sont reliées à l'extrémité distale de la section de gaine proximale (2) par un accouplement (14) conformé de façon à pouvoir recevoir un mors (9') correspondant au mors (9), un entraîneur (16) plus gros étant agencé à l'extrémité de la tige (15) qui dépasse du côté proximal de l'élément d'assemblage (13).
